# EUROPEAN PATENT SPECIFICATION

(11) **EP 2 208 479 B1**
(45) Date of publication and mention of the grant of the patent: **09.05.2012**
(21) Application number: 10394001.1
(22) Date of filing: 15.01.2010
(51) Int. Cl.: A61F 2/01

(54) **A vascular filter**
Gefässfilter
Filtre vasculaire

(30) Priority: 16.01.2009 IE 20090040
(43) Date of publication of application: 21.07.2010
(60) Divisional application: 12075021.1
(73) Proprietor: Novate Medical Ltd., Dublin 2 (IE)
(72) Inventor: Horan, Steven, Galway (IE); O'Gorman, Jacquline, County Clare (IE); Goggin, Aidan, County Donegal (IE)
(74) Representative: Weldon, Michael James

(56) References cited:
- EP-A1- 1 616 530
- WO-A2-01/62184
- WO-A2-2008/010197
- US-A1- 2006 025 852
- US-A1- 2007 203 571
- US-A1- 2008 188 887
- US-A1- 2008 208 245
- US-B1- 6 214 025

## Description

### Introduction

This invention relates to a vascular filter.

WO2008/010197, US2007/203571, US6214025, WO01/62184, EP1616530, US2008/188887, US2006/025852, and US2008/208245 describe various vascular filter arrangements.

### Statements of Invention

According to the invention there is provided a vascular filter as set out in claim 1.

By capturing the thrombus, the filter prevents the thrombus from passing to the heart or lungs, which may cause pulmonary embolism. By supporting the capture members this ensures that the capture members are maintained in the desired location in the blood vessel.

Preferably in the capturing configuration the capture member extends towards an apex. Ideally in the capturing configuration the capture member extends towards the apex in a curve.

In another embodiment the support member is movable between a delivery configuration and a deployed configuration.

Preferably the support member comprises a plurality of connector elements, each connector element connecting a peak of the wave pattern to an adjacent peak of the wave pattern. Ideally the support member comprises an enlarged end element at a peak of the wave pattern. This arrangement results in greater flexibility, in lower strains, and in greater resistance to buckling. Most preferably in the delivery configuration the diameter of curvature of the end element is greater than the distance between adjacent connector elements. The wave pattern of the support member may comprise less than twelve distal peaks. This arrangement results in greater resistance to buckling. Preferably the wave pattern of the support member comprises less than ten distal peaks. Ideally the wave pattern of the support member comprises less than eight distal peaks. The capture member may be connected to the support member at a distal peak of the wave pattern. Preferably a first capture member is connected to the support member at a first distal peak of the wave pattern, and a second capture member is connected to the support member at the first distal peak of the wave pattern. Ideally in the capturing configuration the first capture member extends from the first distal peak of the wave pattern towards the apex in a first curve, and the second capture member extends from the first distal peak of the wave pattern towards the apex in a second curve, the concave portion of the first curve facing inwardly towards the concave portion of the second curve. This arrangement minimises any gap between adjacent capture members, and thus improves filter efficiency.

The invention minimises any bowing or lift-off of the support member from the wall of the blood vessel. Thus the contact force between the support member and the blood vessel is maximised which enhances resistance to buckling Most preferably when the filter is deployed externally of a blood vessel, in the open configuration the convex portion of the curve faces radially outwardly. In the open configuration the ratio of R1:R2 may be in the range of from 1:1 to 1.5:1, where
R1 is the distance of the support member from the central longitudinal axis of the filter at the point along the support member where this distance is at a maximum, and
R2 is the distance of the support member from the central longitudinal axis of the filter at an end of the support member.

In the capturing configuration the maximum distance between the support member and a wall of a blood vessel may be less than 4mm. In the capturing configuration the maximum distance between the support member and a wall of a blood vessel may be less than 2mm.

In one embodiment the radial dimension of the support member is greater than 0.20mm. This arrangement results in greater resistance to buckling. Preferably the radial dimension of the support member is greater than 0.25mm. Ideally the radial dimension of the support member is greater than 0.30mm.

In another embodiment at least part of the support member comprises one or more elongate elements, the width of at least one of the elongate elements being greater than 0.25mm. This arrangement results in greater resistance to buckling. Preferably the width of the elongate element is greater than 0.30mm. Ideally the width of the elongate element is greater than 0.35mm.

### Brief Description of the Drawings

The invention will be more clearly understood from the following description of an embodiment thereof, given by way of example only, with reference to the accompanying drawings, in which:
Fig. 1 is an isometric view of a vascular filter according to the invention in a deployed capturing configuration,
Fig. 2 is a developed side view of the vascular filter of Fig. 1 in a delivery configuration,
Fig. 3 is an enlarged developed side view of part of the vascular filter of Fig. 2 in the delivery configuration,
Fig. 4 is a side view of the vascular filter of Fig. 1 in the deployed capturing configuration,
Fig. 5 is an enlarged side view of part of the vascular filter of Fig. 4 in the deployed capturing configuration,
Fig. 6 is an end view of the vascular filter of Fig. 1 in the deployed capturing configuration,
Fig. 7 is an enlarged end view of part of the vascular filter of Fig. 6 in the deployed capturing configuration,
Fig. 7(a) is an end view of the vascular filter of Fig. 1 during manufactures
Fig. 7(b) is an enlarged end view of part of the vascular filter of Fig. 7(a) during manufacture,
Fig. 8 is a side view of the vascular filter of Fig. 1 in a deployed open configuration,
Fig. 9 is a graph of force versus displacement, and
Fig. 10 is an end view illustrating buckling of a vascular filter.

### Detailed Description

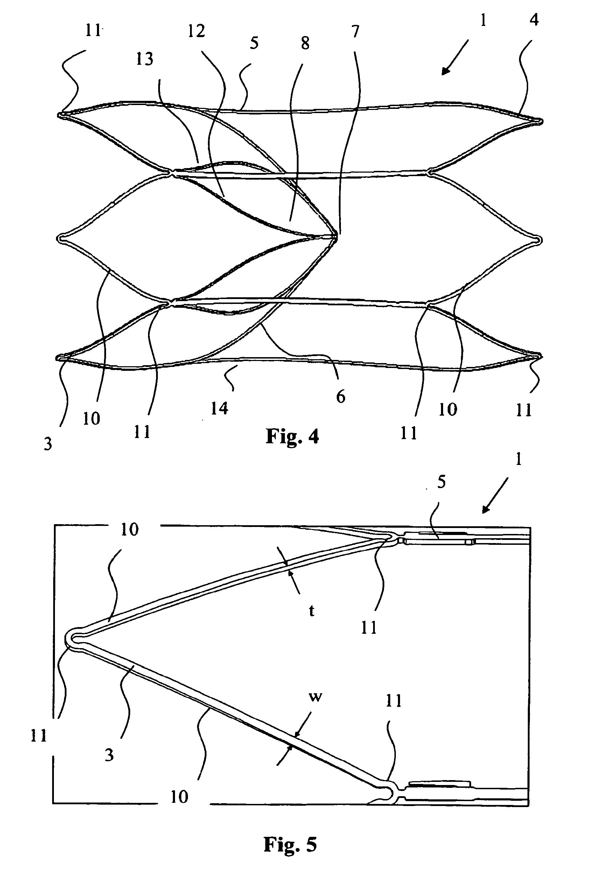

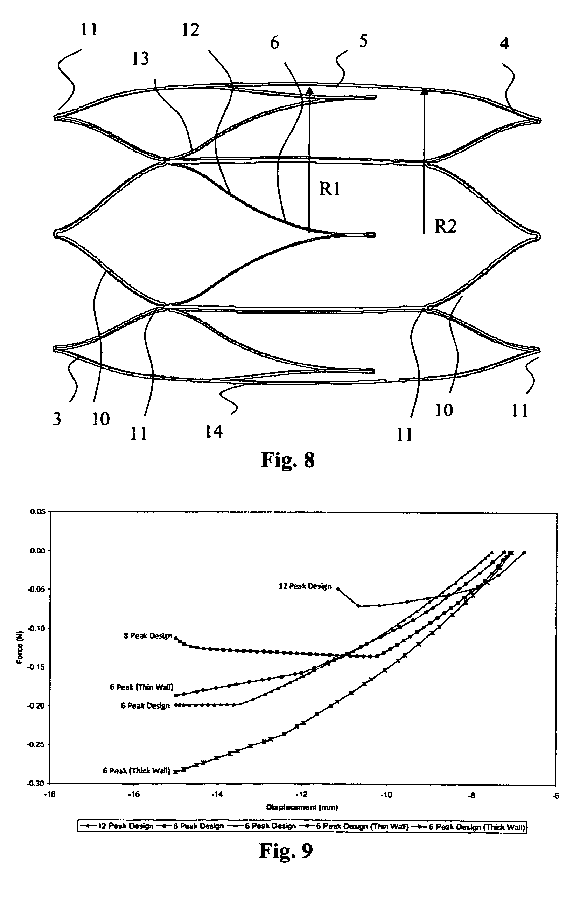

Referring to the drawings there is illustrated a vascular filter 1 according to the invention. The vascular filter 1 is suitable for use as an inferior vena cava filter in the inferior vena cava. The filter 1 is movable from a capturing configuration (Fig. 4) to an open configuration (Fig. 8) upon elapse of a predetermined period of time. In the capturing configuration the filter 1 is configured to capture thrombus passing through the inferior vena cava towards the heart and the lungs. The filter 1 may thus be used to prevent pulmonary embolism. In the open configuration the filter 1 is configured to facilitate unrestricted blood flow.

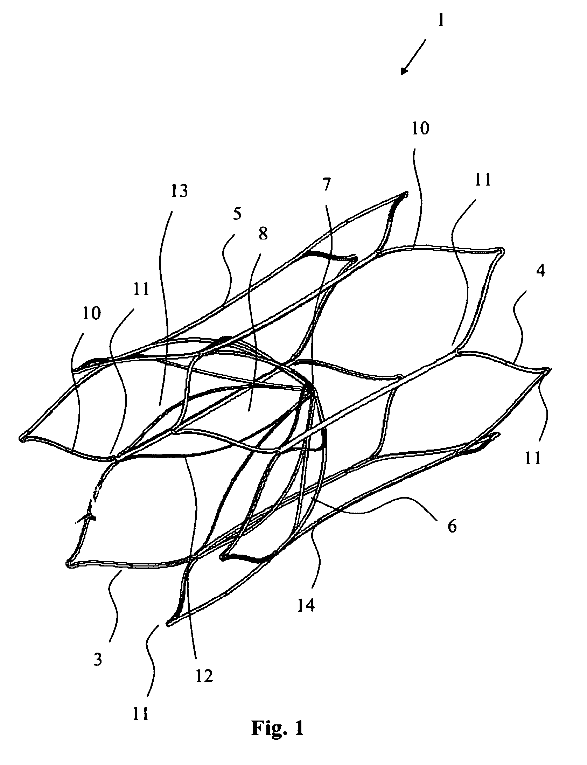

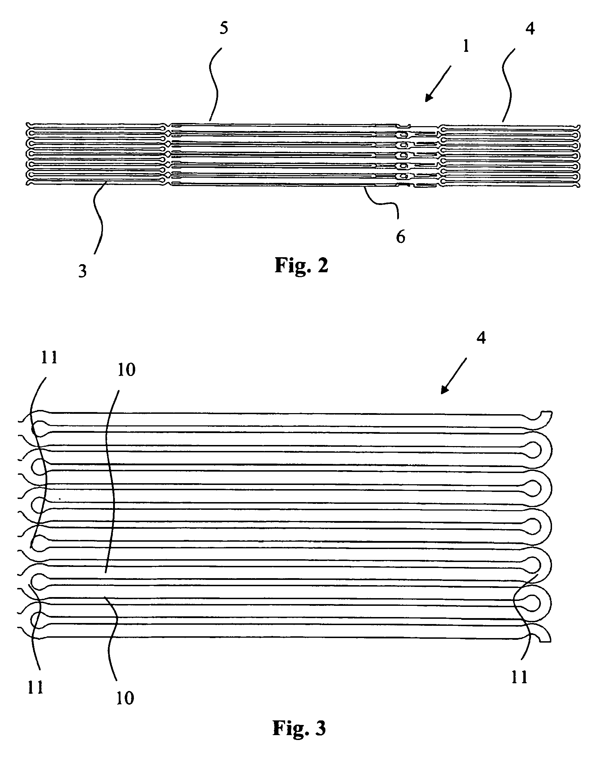

The filter 1 is movable between a collapsed delivery configuration (Figs. 2 and 3) and an expanded deployed configuration (Figs. 1 and 4). The filter 1 is biased radially outwardly towards the deployed configuration.

As illustrated in Fig. 1, the filter 1 comprises a proximal support hoop 3 at the proximal end of the filter 1, a distal support hoop 4 at the distal end of the filter 1, and a plurality of support struts 5 extending between the proximal support hoop 3 and the distal support hoop 4.

In this patent specification, the term 'proximal' will be understood to mean the end closest to a user when carrying out a procedure accessed from a femoral vein, or the caudal end. Similarly the term 'distal' will be understood to mean the end furthest from a user when carrying out a procedure accessed from a femoral vein, or the cranial end.

The proximal support hoop 3 extends circumferentially around the internal wall of the inferior vena cava in a zig-zag wave pattern. As illustrated in Fig. 5, the proximal support hoop 3 comprises a plurality of elongate connector elements 10. Each connector element 10 connects a peak 11 of the wave pattern to an adjacent peak 11 of the wave pattern.

In this case the wave pattern of the proximal support hoop 3 comprises six distal peaks 11 and six proximal peaks 11. It has been found that the filter 1 having six distal peaks 11 and six proximal peaks 11 is particularly resistant to buckling.

The proximal support hoop 3 comprises an enlarged end element at each peak 11 of the wave pattern. In the delivery configuration the diameter of curvature of the end element 11 is greater than the distance between adjacent connector elements 10. The geometry of the crown tips 11 allows for less rigid movement of the proximal support hoop 3 and also allows for lower strains in the proximal support hoop 3. Enhanced flexibility of the crown 3 minimises the probability of buckling. The crown 3 of the filter 1 offers increased torsional flexibility. This flexibility helps to minimise the probability of buckling.

As illustrated in Fig. 5, the radial dimension or wall thickness t of the proximal support hoop 3 is 0.33mm in this case. It has been found that the filter 1 has a greater resistance to buckling and a higher radial force. The width w of each connector element 10 is 0.38mm in this case. The filter 1 with six distal peaks 11 and six proximal peaks 11has a particularly low profile in the delivery configuration. This low profile enables larger strut thickness t and width w for a given delivery profile.

Similarly the distal support hoop 4 extends circumferentially around the internal wall of the inferior vena cava in a zig-zag wave pattern. The distal support hoop 4 comprises a plurality of elongate connector elements 10. Each connector element 10 connects a peak 11 of the wave pattern to an adjacent peak 11 of the wave pattern.

In this case the wave pattern of the distal support hoop 4 comprises six distal peaks 11 and six proximal peaks 11.

The distal support hoop 4 comprises an enlarged end element at each peak 11 of the wave pattern. As illustrated in Fig. 3, in the delivery configuration the diameter of curvature of the end element 11 is greater than the distance between adjacent connector elements 10.

The radial dimension or wall thickness of the distal support hoop 4 is 0.33mm in this case. The width of each connector element 10 is 0.38mm in this case.

The support struts 5 extend longitudinally along the internal wall of the inferior vena cava in a curve 14. The support struts 5 connect the proximal support hoop 3 to the distal support hoop 4. In this case the proximal support hoop 3, the distal support hoop 4 and the support struts 5 are formed integrally. The proximal support hoop 3, the distal support hoop 4 and the support struts 5 may be of a shape-memory material, such as Nitinol™.

The radial dimension or wall thickness of each support strut 5 is 0.33mm in this case. The width of each support strut 5 is 0.38mm in this case.

As illustrated in Fig. 1, the filter 1 comprises twelve capture arms 6 for capturing thrombus passing through the inferior vena cava.

Each capture arm 6 is formed integrally with the proximal support hoop 3. As illustrated in Fig. 4, for each distal peak 11 of the wave pattern a first capture arm 6 is connected to the proximal support hoop 3 at the distal peak 11, and a second capture arm 6 is connected to the proximal support hoop 3 at the distal peak 11.

Each capture arm 6 is movable from the capturing configuration (Fig. 4) to the open configuration (Fig. 8) upon elapse of the predetermined period of time. In the capturing configuration the capture arms 6 are configured to capture thrombus passing through the inferior vena cava towards the heart and the lungs. In the open configuration the capture arms 6 are configured to facilitate unrestricted blood flow.

In the capturing configuration each capture arm 6 extends to an apex 7 in a curve. As illustrated in Fig. 4, for each distal peak 11 of the wave pattern the first capture arm 6 extends from the distal peak 11 to the apex 7 in a first curve 12, and the second capture arm 6 extends from the distal peak 11 to the apex 7 in a second curve 13. The concave portion of the first curve 12 faces inwardly towards the concave portion of the second curve 13. In this manner the capture arms 6 define a generally conically shaped capture region 8 within which thrombus may be captured. Any gaps between adjacent filter elements 6 in the filtration cone 8 are minimised. The filter elements 6 are shape set to maximise filter efficiency.

In this specification, the term curve will be understood to mean a smooth curve or two or more discreet straight sections. For example, the filter element 6 curvature may be heat set to extend towards the apex in a series of two straights. It is appreciated that 2 or more discrete straights, or, a smooth curve, or, a set of smooth curves, may be employed to maximise filter efficiency.

A perimeter route is defined from a first distal peak 11 of the proximal support hoop 3 along a first connector element 10 to a proximal peak 11 of the proximal support hoop 3, from the proximal peak 11 along a second connector element 10 to a second distal peak 11 of the proximal support hoop 3, from the second distal peak 11 along a first support strut 5 to a first proximal peak 11 of the distal support hoop 4, from the first proximal peak 11 along a third connector element 10 to a distal peak 11 of the distal support hoop 4, from the distal peak 11 along a fourth connector element 10 to a second proximal peak 11 of the distal support hoop 4, from the second proximal peak 11 along a second support strut 5 to the first distal peak 11 of the proximal support hoop 3. A cell is defined within the perimeter route. The filter 1 comprises six such cells. Two capture arms 6 are attached to each cell in both the capturing configuration (Fig. 4) and the open configuration (Fig. 8). This arrangement results in a balanced cell spacing for consistent filtration pore size.

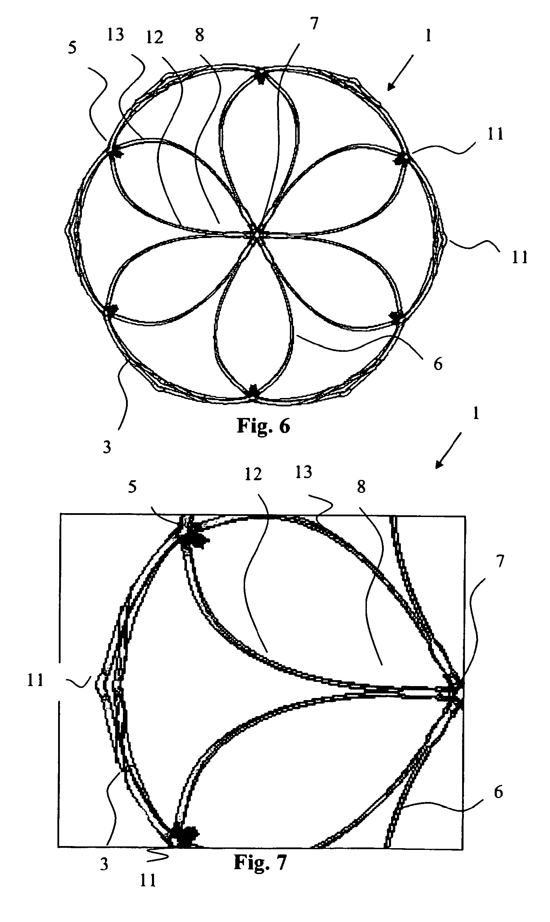

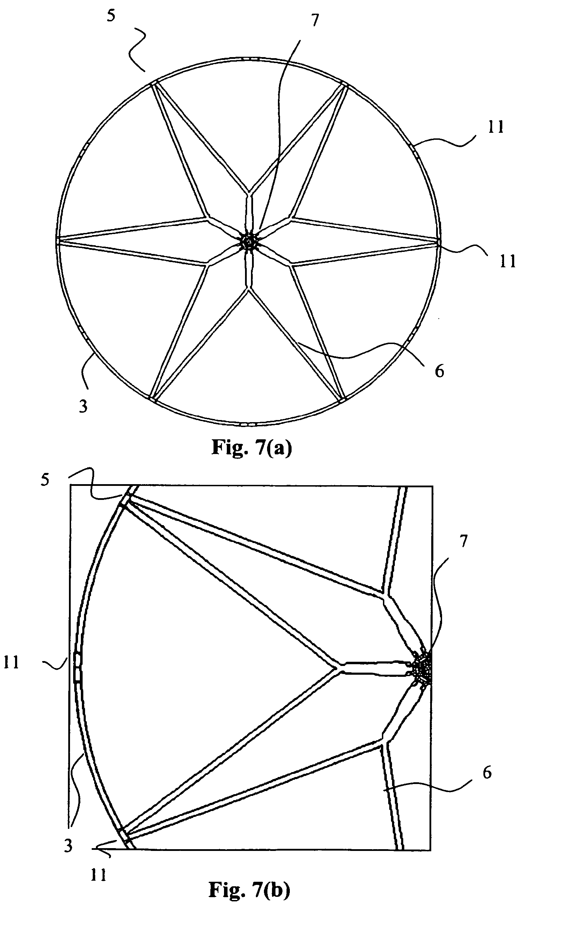

During manufacture the capture arms 6 are formed into the curved shapes. Figs. 7(a) and 7(b) illustrate the capture arms 6 before forming into the curved shapes. Figs. 7(a) and 7(b) illustrate the cut pattern of Fig. 2 expanded without heat setting the capture arms 6.

When the filter 1 is deployed in the inferior vena cava, the apex 7 is substantially in-line with the longitudinal axis extending through the centre of the inferior vena cava, and the capture region 8 is located in the region of the centre of the inferior vena cava. When the filter 1 is deployed in the inferior vena cava, the capture arms 6 extend in the direction of blood flow through the inferior vena cava.

The capture arms 6 are movable from the capturing configuration to the open configuration upon elapse of the predetermined period of time. The capture arms 6 are biased towards the open configuration.

The filter 1 comprises a holder member at the distal ends of the capture arms 6 to temporarily hold the capture arms 6 in the capturing configuration until elapse of the predetermined period of time. The holder member engages with each capture arm 6 to hold the capture arms 6 in the capturing configuration. At least part of the holder member is biodegradable and/or bioabsorbable upon elapse of the predetermined period of time. Upon biodegrading/bioabsorbing of the holder member, the capture arms 6 are free to move from the capturing configuration to the open configuration. The capture arms 6 are not biodegradable or bioabsorbable.

The distal end of the distal support hoop 4 is located distally of the capture arms 6 and the apex 7, and the proximal end of the proximal support hoop 3 is located proximally of the capture arms 6.

When the filter 1 is deployed in the inferior vena cava, the support hoops 3, 4 and the support struts 5 exert a force radially outwardly on the internal wall of the inferior vena cava. In this manner the support hoops 3, 4 and the support struts 5 support the capture arms 6 in position relative to the wall of the inferior vena cava 2.

In the capturing configuration the maximum distance between each support strut 5 and a wall of a blood vessel may be less than 4mm, and preferably is less than 2mm. In the capturing configuration the concave portion of the curve 14 of the support struts 5 may face radially outwardly (Fig. 4), or alternatively the support struts 5 may be straight, or alternatively the convex portion of the curve 14 of the support struts 5 may face radially outwardly.

As illustrated in Fig. 8, when the filter is deployed externally of a blood vessel, in the open configuration the convex portion of the curve 14 of the support struts 5 faces radially outwardly. In the open configuration the ratio of R1:R2 is in the range of from 1:1 to 1.5:1, where

R1 is the distance of each support strut 5 from the central longitudinal axis of the filter 1 at the point along the support strut 5 where this distance is at a maximum, and

R2 is the distance of the support strut 5 from the central longitudinal axis of the filter 1 at an end of the support strut 5 where the support strut 5 is connected to either the proximal support hoop 3 or the distal support hoop 4.

In this case the largest radius R1 of the convex portion of the curve 14 is 17mm, and the radius R2 of the proximal support hoop 3 and the distal support hoop 4 is 15mm. The barrel shape of the filter 1 reduces any bowing or lift-off of the support struts 5 from the blood vessel wall, increasing the contact force of the support hoops 3, 4 with the blood vessel, and improving buckling resistance.

In use the filter 1 is collapsed to the delivery configuration (Figs. 2 and 3), and at least partially loaded into a delivery catheter. The delivery catheter is advanced through the inferior vena cava until the collapsed filter 1 reaches the desired location in the inferior vena cava. A restraining sheath of the delivery catheter is then moved proximally relative to the filter 1 to fully uncover the filter 1. Due to the biasing nature of the filter 1, the filter 1 moves from the collapsed delivery configuration to the expanded deployed configuration (Fig. 4). In the deployed configuration, the support hoops 3, 4 and the support struts 5 exert a radially outward force on the internal wall of the inferior vena cava to support the capture arms 6 in the desired position in the inferior vena cava.

In the event of thrombus passing through the inferior vena cava towards the heart and the lungs, the thrombus will be captured in the capture region 8 of the filter 1. The thrombus will thus be prevented from passing into the heart and the lungs which could otherwise lead to pulmonary embolism. The captured thrombus will gradually be broken down by the body into smaller size particles which will significantly reduce the risk of embolism.

The holder member temporarily holds the capture arms 6 in the capturing configuration until elapse of the predetermined period of time. Upon elapse of the predetermined period of time the holder member biodegrades/bioabsorbs. This enables the capture arms 6 to move from the capturing configuration to the open configuration (Fig. 8). In the open configuration the filter 1 facilitates unrestricted blood flow. The support hoops 3, 4, the support struts 5 and the capture arms 6 remain in the inferior vena cava.

It will be appreciated that the proximal support hoop 3 and/or the distal support hoop 4 may comprise any suitable number of distal peaks and proximal peaks. For example the proximal support hoop 3 and/or the distal support hoop 4 may comprise eleven distal peaks and eleven proximal peaks, or may comprise ten distal peaks and ten proximal peaks, or may comprise nine distal peaks and nine proximal peaks, or may comprise eight distal peaks and eight proximal peaks, or may comprise seven distal peaks and seven proximal peaks, or may comprise five distal peaks and five proximal peaks, or may comprise four distal peaks and four proximal peaks, or may comprise three distal peaks and three proximal peaks.

Fig. 9 illustrates the buckling behaviour of filters having a different number of peaks. Each filter was placed in a blood vessel having a 16mm diameter and a crown tip 11 was pulled toward the centre of the blood vessel. The reaction force or force exerted by the filter against this movement is plotted on the graph of Fig. 9. The force and displacement values on the graph are negative as the force is a compressive/pushing force and the crown tip 11 is moving negatively into the centre of the blood vessel.

Fig. 9 illustrates that at the largest displacement the filter having six distal peaks and six proximal peaks exerts a particularly high force against the deformation applied, and thus the filter having six distal peaks and six proximal peaks is highly resistant to buckling.

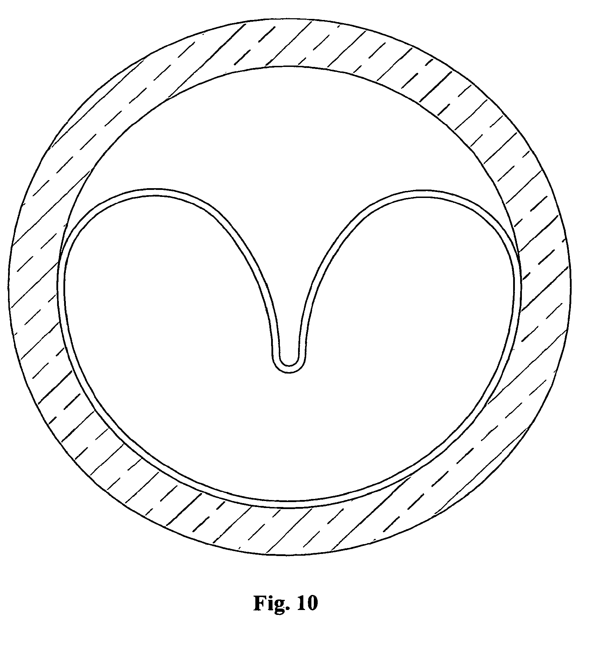

Fig. 10 illustrates buckling of a vascular filter deployed in a blood vessel.

The invention is not limited to the embodiment hereinbefore described, with reference to the accompanying drawings, which may be varied in construction and detail.

## Claims

1. A vascular filter (1) comprising:
capture members (6) for capturing thrombus passing through a blood vessel, and
support members (3, 4, 5) for supporting the capture members relative to a wall of the blood vessel;
wherein the support members (3, 4, 5) are movable between a delivery configuration and a deployed configuration;
wherein at least some of (10) the support members are configured to extend circumferentially around a wall of a blood vessel in a wave pattern,
wherein the capture members (6) are movable from a capturing configuration to an open configuration, in the capturing configuration the capture member being configured to capture thrombus passing through a blood vessel, in the open configuration the capture member being configured to facilitate unrestricted blood flow;
wherein
the support members comprise a proximal hoop in a wave pattern, a distal hoop in a wave pattern, and struts extending longitudinally along the wall of a blood vessel interconnecting the proximal and distal hoops, and capture members are connected to the support members at or adjacent to distal peaks of the proximal hoop; **characterized in that**
the struts have a convex curve when the capture members are in the open configuration and the struts have a concave curve when the capture members are in the capturing configuration, due to action of the capture members on the support member.

2. A filter as claimed in claim 1, wherein the capture members (6) extend towards an apex in curves in which a concave portion of a curve of a first capture member faces towards the concave portion of a curve of a second capture member.

3. A filter as claimed in any preceding claim, wherein the support members comprises a plurality of connector elements (4, 10), each connector element connecting a peak of the wave pattern to an adjacent peak of the wave pattern.

4. A filter as claimed in any preceding claim, wherein the support members comprise an enlarged end element (11) at a peak of the wave pattern.

5. A filter as claimed in claim 4, wherein in the delivery configuration the diameter of curvature of the end element (11) is greater than the distance between adjacent connector elements.

6. A filter as claimed in any preceding claim, wherein the wave pattern of the support members comprises less than eight distal peaks.

7. A filter as claimed in claim 6, wherein the wave pattern comprises six peaks.

8. A filter as claimed in any preceding claim, wherein a first capture member is connected to the support members at or adjacent to a first distal peak of the proximal hoop, and a second capture member is also connected at or adjacent to the first distal peak of the proximal hoop.

9. A filter as claimed in any preceding claim, wherein the strut is configured to extend longitudinally in a curve.

10. A filter as claimed in any preceding claim, wherein in the open configuration the ratio of R1:R2 is in the range of from 1:1 to 1.5:1, where
R1 is the distance of the support member from the central longitudinal axis of the filter at the point along the support member where this distance is at a maximum, and
R2 is the distance of the support member from the central longitudinal axis of the filter at an end of the support member.

11. A filter as claimed in any preceding claim, wherein in the capturing configuration the maximum distance between the support member and a wall of a blood vessel is less than 4mm.

12. A filter as claimed in claim 11 wherein in the capturing configuration the maximum distance between the support member and a wall of a blood vessel is less than 2mm.

13. A filter as claimed in any of claims 1 to 12 wherein the radial dimension of the support members is greater than 0.20mm.

14. A filter as claimed in claim 13 wherein the radial dimension of the support members is greater than 0.25mm.

15. A filter as claimed in claim 14 wherein the radial dimension of the support members is greater than 0.30mm.

16. A filter as claimed in any of claims 1 to 15 wherein at least part of the support member comprises one or more elongate elements, the width of at least one of the elongate elements being greater than 0.25mm.

17. A filter as claimed in claim 16 wherein the width of the elongate element is greater than 0.30mm.

18. A filter as claimed in claim 17 wherein the width of the elongate element is greater than 0.35mm.

19. A filter as claimed in any preceding claim, wherein the capture members extend between the plurality of struts when in the open configuration.

## Patentansprüche

1. Gefäßfilter (1), der Folgendes umfasst:
Einfangelemente (6) zum Einfangen eines ein Blutgefäß durchquerenden Thrombus, und
Abstützelemente (3, 4, 5) zum Abstützen der Einfangelemente in Bezug zu einer Wand des Blutgefäßes;
wobei die Abstützelemente (3, 4, 5) zwischen einer Zuführkonfiguration und einer entfalteten Konfiguration bewegt werden können;
wobei mindestens einige (10) der Abstützelemente konfiguriert sind, um sich in einem Wellenmuster am Umfang um eine Wand eines Blutgefäßes herum zu erstrecken;
wobei die Einfangelemente (6) aus einer Einfangkonfiguration zu einer offenen Konfiguration bewegt werden können, das Einfangelement in der Einfangkonfiguration konfiguriert ist, um einen das Blutgefäß durchquerenden Thrombus einzufangen, und das Einfangelement in der offenen Konfiguration konfiguriert ist, um ungehinderten Blutfluss zu vereinfachen;
wobei
die Abstützelemente einen proximalen Ring in einem Wellenmuster, einen distalen Ring in einem Wellenmuster, und Streben aufweisen, die sich in Längsrichtung entlang der Wand eines Blutgefäßes erstrecken und den proximalen und distalen Ring miteinander verbinden, und die Einfangelemente an oder angrenzend an distale Spitzen des proximalen Rings mit den Abstützelementen verbunden sind; **dadurch gekennzeichnet, dass**
aufgrund von Einwirkung der Einfangselemente auf das Abstützelement, die Streben eine konvexe Krümmung haben, wenn sich die Einfangelemente in der offenen Konfiguration befinden, und die Streben eine konkave Krümmung aufweisen, wenn sich die Einfangelemente in der Einfangkonfiguration befinden.

2. Filter nach Anspruch 1, bei dem sich die Einfangelemente (6) in Richtung eines Scheitels in Krümmungen erstrecken, in denen ein konkaver Teil einer Krümmung eines ersten Einfangelements in Richtung des konkaven Teils einer Krümmung eines zweiten Einfangelements gerichtet ist.

3. Filter nach einem vorhergehenden Anspruch, bei dem die Abstützelemente eine Mehrzahl von Verbindungselementen (4, 10) aufweisen, wobei jedes Verbindungselement eine Spitze des Wellenmusters mit einer angrenzenden Spitze des Wellenmusters verbindet.

4. Filter nach einem vorhergehenden Anspruch, bei dem die Abstützelemente ein vergrößertes Endelement (11) an einer Spitze des Wellenmusters aufweisen.

5. Filter nach Anspruch 4, bei dem in der Zuführkonfiguration der Krümmungsdurchmesser des Endelements (11) größer als der Abstand zwischen benachbarten Verbindungselementen ist.

6. Filter nach einem vorhergehenden Anspruch, bei dem das Wellenmuster der Abstützelemente weniger als acht distale Spitzen aufweist.

7. Filter nach Anspruch 6, bei dem das Wellenmuster sechs Spitzen aufweist.

8. Filter nach einem vorhergehenden Anspruch, bei dem ein erstes Einfangelement mit den Abstützelementen an oder angrenzend an eine erste distale Spitze des proximalen Rings verbunden ist, und ein zweites Einfangelement auch an oder angrenzend an die erste distale Spitze des proximalen Rings verbunden ist.

9. Filter nach einem vorhergehenden Anspruch, bei dem die Strebe konfiguriert ist, um sich längs in einer Krümmung zu erstrecken.

10. Filter nach einem vorhergehenden Anspruch, bei dem in der offenen Konfiguration das Verhältnis von R1:R2 im Bereich von 1:1 bis 1,5:1 liegt; wobei
R1 der Abstand des Abstützelements von der zentralen Längsachse des Filters an dem Punkt entlang des Abstützelements ist, wo dieser Abstand maximal ist, und
R2 der Abstand des Abstützelements von der zentralen Längsachse des Filters an einem Ende des Abstützelements ist.

11. Filter nach einem vorhergehenden Anspruch, bei dem in der Einfangkonfiguration der maximale Abstand zwischen dem Abstützelement und einer Wand eines Blutgefäßes weniger als 4 mm beträgt.

12. Filter nach Anspruch 11, bei dem in der Einfangkonfiguration der maximale Abstand zwischen dem Abstützelement und einer Wand eines Blutgefäßes weniger als 2 mm beträgt.

13. Filter nach einem der Ansprüche 1 bis 12, bei dem die radiale Abmessung der Abstützelemente größer als 0,20 mm ist.

14. Filter nach Anspruch 13, bei dem die radiale Abmessung der Abstützelemente größer als 0,25 mm ist.

15. Filter nach Anspruch 14, bei dem die radiale Abmessung der Abstützelemente größer als 0,30 mm ist.

16. Filter nach einem der Ansprüche 1 bis 15, bei dem zumindest ein Teil des Abstützelements ein oder mehrere Langelemente aufweist, und die Breite mindestens eines der Langelemente größer als 0,25 mm ist.

17. Filter nach Anspruch 16, bei dem die Breite des Langelements größer als 0,30 mm ist.

18. Filter nach Anspruch 17, bei dem die Breite des Langelements größer als 0,35 mm ist.

19. Filter nach einem vorhergehenden Anspruch, bei dem sich die Einfangelemente in der offenen Konfiguration zwischen der Anzahl von Streben erstrecken.

## Revendications

1. Filtre vasculaire (1) comportant :
des éléments de capture (6) destinés à capturer des thrombus passant au travers d'un vaisseau sanguin, et
des éléments de support (3, 4, 5) destinés à supporter les éléments de capture par rapport à une paroi du vaisseau sanguin ;
dans lequel les éléments de support (3, 4, 5) sont mobiles entre une configuration d'acheminement et une configuration déployée ;
dans lequel au moins certains des (10) éléments de support sont configurés pour s'étendre de manière circonférentielle autour d'une paroi d'un vaisseau sanguin en une forme de vague,
dans lequel les éléments de capture (6) sont mobiles d'une configuration de capture en une configuration ouverte, dans la configuration de capture l'élément de capture étant configuré pour capturer des thrombus passant au travers d'un vaisseau sanguin, dans la configuration ouverte l'élément de capture étant configuré pour faciliter un débit sanguin libre ;
dans lequel
les éléments de support comportent un cerceau proximal en une forme de vague, un cerceau distal en une forme de vague, et des entretoises s'étendant dans le sens longitudinal le long de la paroi d'un vaisseau sanguin raccordant ensemble le cerceau proximal et le cerceau distal, et des éléments de capture sont raccordés aux éléments de support au niveau de ou de manière adjacente à des crêtes distales du cerceau proximal ; **caractérisé en ce que** les entretoises ont une courbe convexe quand les éléments de capture sont dans la configuration ouverte et les entretoises ont une courbe concave quand les éléments de capture sont dans la configuration de capture, en raison de l'action des éléments de capture sur l'élément de support.

2. Filtre selon la revendication 1, dans lequel les éléments de capture (6) s'étendent vers un sommet sous la forme de courbes dans lesquelles un partie concave d'une courbe d'un premier élément de capture est orientée vers la partie concave d'une courbe d'un second élément de capture.

3. Filtre selon l'une quelconque des revendications précédentes, dans lequel les éléments de support comportent une pluralité d'éléments de raccordement (4, 10), chaque élément de raccordement raccordant une crête de la forme de vague à une crête adjacente de la forme de vague.

4. Filtre selon l'une quelconque des revendications précédentes, dans lequel les éléments de support comportent un élément d'extrémité agrandi (11) au niveau d'une crête de la forme de vague.

5. Filtre selon la revendication 4, dans lequel, dans la configuration d'acheminement, le diamètre de courbure de l'élément d'extrémité (11) est supérieur à la distance entre des élément de raccordement adjacents.

6. Filtre selon l'une quelconque des revendications précédentes, dans lequel la forme de vague des éléments de support comporte moins de huit crêtes distales.

7. Filtre selon la revendication 6, dans lequel la forme de vague comporte six crêtes.

8. Filtre selon l'une quelconque des revendications précédentes, dans lequel un premier élément de capture est raccordé aux éléments de support au niveau d'une ou de manière adjacente par rapport à une première crête distale du cerceau proximal, et un second élément de capture est également raccordé au niveau de la ou de manière adjacente par rapport à la première crête distale du cerceau proximal.

9. Filtre selon l'une quelconque des revendications précédentes, dans lequel l'entretoise est configurée pour s'étendre dans le sens longitudinal sous la forme d'une courbe.

10. Filtre selon l'une quelconque des revendications précédentes, dans lequel, dans la configuration ouverte, le rapport de R1:R2 se situe dans la gamme allant de 1:1 à 1,5:1, où
R1 est la distance de l'élément de support depuis l'axe longitudinal central du filtre au niveau du point le long de l'élément de support où cette distance est à un maximum, et
R2 est la distance de l'élément de support depuis l'axe longitudinal central du filtre au niveau d'une extrémité de l'élément de support.

11. Filtre selon l'une quelconque des revendications précédentes, dans lequel, dans la configuration de capture, la distance maximale entre l'élément de support et une paroi d'un vaisseau sanguin est inférieure à 4 mm.

12. Filtre selon la revendication 11, dans lequel, dans la configuration de capture, la distance maximale entre l'élément de support et une paroi d'un vaisseau sanguin est inférieure à 2 mm.

13. Filtre selon l'une quelconque des revendications 1 à 12, dans lequel la dimension radiale des éléments de support est supérieure à 0,20 mm.

14. Filtre selon la revendication 13, dans lequel la dimension radiale des éléments de support est supérieure à 0,25 mm.

15. Filtre selon la revendication 14, dans lequel la dimension radiale des éléments de support est supérieure à 0,30 mm.

16. Filtre selon l'une quelconque des revendications 1 à 15, dans lequel au moins une partie de l'élément de support comporte un ou plusieurs éléments allongés, la largeur d'au moins l'un des éléments allongés étant supérieure à 0,25 mm.

17. Filtre selon la revendication 16, dans lequel la largeur de l'élément allongé est supérieure à 0,30 mm.

18. Filtre selon la revendication 17, dans lequel la largeur de l'élément allongé est supérieure à 0,35 mm.

19. Filtre selon l'une quelconque des revendications précédentes, dans lequel les éléments de capture s'étendent entre la pluralité d'entretoises lorsque dans la configuration ouverte.
